# EUROPEAN PATENT SPECIFICATION

(11) **EP 4 021 337 B1**
(45) Date of publication and mention of the grant of the patent: **06.03.2024**
(21) Application number: 20765365.0
(22) Date of filing: 26.08.2020
(51) Int. Cl.: A61B 90/00, A61B 17/34

(54) **TISSUE ANCHOR**
GEWEBEANKER
ANCRAGE DE TISSU

(30) Priority: 26.08.2019 US 201962891546 P
(43) Date of publication of application: 06.07.2022
(73) Proprietor: Janssen Biotech, Inc., Horsham, PA 19044 (US)
(72) Inventor: DAGLOW, Terry, Houston, Texas 77042 (US)
(74) Representative: Carpmaels & Ransford LLP
(86) International application number: PCT/IB2020/057991
(87) International publication number: WO 2021/038474

(56) References cited:
- US-A- 5 755 732
- US-A- 5 879 357
- US-A1- 2011 282 176
- US-A1- 2015 045 665
- US-A1- 2015 057 531
- US-A1- 2016 166 278
- US-A1- 2016 206 338
- US-B1- 6 482 178

## Description

### TECHNICAL FIELD

The present invention relates to tissue anchors. More particularly, the present invention relates to tissue anchors for anchoring catheters or other access ports within tissue.

### BACKGROUND ART

The current methods for diagnosing and/or treating tumors and lesions frequently employ image guided devices, e.g., needles. Currently such devices require an external stabilization apparatus to maintain the desired target location, e.g., lesion or tumor, and continued re-imaging to confirm the target location has not changed which sometimes happens due to breathing or other anatomical movement.

Various devices are known in the art that include tissue anchoring mechanisms. For example, U.S. Patent No. 8,888,798 discloses a tissue repair device including an anchor disposed within the lumen of a needle. An actuator is disposed within the needle that includes a distal end engaged with the anchor and a proximal end coupled to a pusher shaft. A knob is provided for advancing the pusher shaft to deploy the anchor.

U.S. Patent No. 8,956,318 discloses a gastrointestinal bypass device having a tissue anchor. The tissue anchor includes a proximal retention element, a distal retention element, and a tension element. The proximal retention element is configured to be deployed on a proximal side of a tissue wall and may take the form of a button or a bar. The distal retention element is configured to be deployed on a distal side of a tissue wall. The distal retention element includes a hub and a plurality of petals. An anchor delivery device is provided that includes a handle, a catheter, a side port, a sled, a delivery needle, a pushrod and a holder. The pushrod may be configured to push out a tissue anchor loaded in the delivery needle.

U.S. Patent Publication No. 2015/0366556 A1 discloses a system and method for anchoring an implant. An anchor, which may comprise a shape-set shape memory material, is deliverable from an inner lumen of a needle or introducer. The needle can be advanced distally below a tissue surface. A pusher can be used to expose the anchor. The pusher and needle can then be retracted once the anchor is retained within the tissue.

International Patent Publication No. WO 2019102484 A1 discloses a multiple anchor delivery system including an anchor loaded in a hollow needle and a driving assembly for deploying the anchor. The driving assembly includes a pusher which may be advanced and retracted by an actuator such as a trigger or a roller.

International Patent Publication No. WO 2020051147 A1 is directed to tissue anchors and needles for tissue anchor deployment. A tissue anchor is disclosed disposed within a delivery system. The delivery system includes a shaft and a needle disposed within a lumen of the shaft. The anchor is disposed within a lumen of the needle. A pusher/ejector is slidably disposed in the needle lumen and may be configured to push the tissue anchor out of a distal end of the needle. US2015045665 (A1) describes 3-dimensional simultaneous multiple core biopsy or fiducial marker placement devices and methods. US2016166278 (A1) describes a dilator for accessing a joint space.

Further improvements are still needed, however, for a more efficient anchored catheter or access port that results in a uniform and continuous deployment which will achieve precise positioning thus minimize or eliminate tissue cutting or tearing.

### SUMMARY OF THE INVENTION

The apparatus of the current invention as defined by claim 1, may be deployed in or around a tumor or lesion in a patient and provide a pathway for subsequent therapeutic or diagnostic minimally invasive surgical procedures or a fluid delivery pathway within the central lumen, i.e. access port. Further embodiments of the invention are defined by the dependent claims. Methods are not claimed per se.

In at least one embodiment, an apparatus according to the invention includes a housing and a wheel disposed in and protruding from the housing. A threaded rod is engaged with and is attached to the wheel, the rod includes a first threaded section and a second threaded section. The threaded rod may be extended through the center or off-of-the center of the wheel. A needle casing is disposed within the housing and is engaged with the first threaded section of said threaded rod at a first end. An anchor casing is disposed within the housing. The anchor casing is engaged with the second threaded section of said threaded rod at a first end. A needle is disposed and secured in the needle casing. An anchor assembly includes a tube having a proximal end and distal end including a plurality of anchor members extending therefrom. In yet at least one other embodiment, an apparatus according to the invention includes a housing and a wheel disposed in and protruding from the housing. A threaded rod engages with and extends through the wheel. The threaded rod includes a first threaded section and a second threaded section. A needle casing is disposed at least in part within the housing and the needle casing is engaged with the first threaded section of the threaded rod. An anchor casing is disposed at least in part within the housing. The anchor casing is engaged with the second threaded section of the threaded rod. A needle is disposed in the needle casing. The needle includes a proximal end and a beveled distal end having a bevel angle. An anchor assembly is provided and includes a tube having a proximal end and distal end and an anchoring device attached to the distal end of the tube. The anchor assembly extends through the needle, the needle housing, and the threaded rod and has a proximal end connected to the anchor casing.

In at least another embodiment of the current invention, an apparatus includes a housing and a wheel disposed in and protruding from the housing. A threaded rod is engaged with and is attached to the wheel, the rod includes a first threaded section and a second threaded section and an anchor lumen. The threaded rod may be extended through the center or off-of-the center of the wheel. A needle casing is disposed within the housing. The needle casing is engaged with the first threaded section of the threaded rod at a first end. An anchor casing is disposed within the housing. The anchor casing is engaged with the second threaded section of said threaded rod at a first end. A needle is disposed and secured in the needle casing. An anchor assembly extends through the anchor lumen of the needle, the needle housing, the threaded rod and is securely attached to the anchor casing. An anchor assembly includes a tube having a proximal end and distal end including a plurality of anchor members extending therefrom. A fluid delivery assembly is provided and includes a tubular shaft that extends distally through the needle and extends proximally through the anchor casing. The tubular shaft is configured to move axially relative to said needle.

In operation, the needle is advanced to a desired target via the use of any known image guided procedure. Once the desired position of the medical device disclosed herein has been achieved, the user rotates the wheel, the needle will be retracted as the tube is translated forward, resulting in a uniform and continuous positioning of the anchoring members.

The position of each anchoring member has been carefully aligned and positioned with respect to the needle bevel angled configuration, resulting in a uniform and continuous deployment. This uniform and continuous deployment will minimize or eliminate tissue cutting or tearing.

Many modifications and variations of the present disclosure may be made without departing from the scope thereof. Therefore, the exemplary embodiments described above should not be used to limit the scope of the invention.

### BRIEF DESCRIPTION OF THE DRAWINGS

**Figure 1** shows a perspective view of the apparatus in accordance with an embodiment of the invention.
**Figure 2** illustrates an exploded view of the apparatus of Figure 1.
**Figure 3A** depicts the apparatus of Figure 1 with a fully retracted anchoring device.
**Figure 3B** shows an exploded view of a needle tip of Figure 3A with the anchoring device disposed therein.
**Figure 4A** illustrates the apparatus of Figure 1 with an anchoring device in a preliminary stage of deployment.
**Figure 4B** depicts an exploded view of the anchoring device of Figure 4A in a preliminary stage of deployment.
**Figure 5A** shows the apparatus of Figure 1 with an anchoring device in an intermediate stage of deployment.
**Figure 5B** portrays an exploded view of the anchoring device of Figure 5A in an intermediate stage of deployment.
**Figure 6A** illustrates the apparatus of Figure 1 with an anchoring device fully deployed.
**Figure 6B** depict an exploded view of the anchoring device of Figure 6A fully deployed.
**Figures 7A and 7B** show a perspective views of an anchoring device according to an embodiment of the invention.

### DESCRIPTION OF EMBODIMENTS

In the following description, certain specific details are set forth in order to provide a thorough understanding of various embodiments of the invention. However, one skilled in the relevant art will recognize that the invention may be practiced without one or more of these specific details, or with other methods, components, materials, etc.

When introducing elements of the current invention or the preferred embodiment(s) thereof, the articles "a", "an", and "the" are intended to mean that there are one or more of the elements.

The term "plurality" when used herein refers to one or more.

Additionally, unless the context requires otherwise, throughout the specification and claims which follow, the word "comprise" and variations thereof, such as, "comprises" and "comprising" are to be construed in an open, inclusive sense that is as "including, but not limited to."

Reference throughout this specification to "one embodiment" or "an embodiment" means that a particular feature, structure or characteristic described in connection with the embodiment is included in at least one embodiment of the present invention. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

As used herein, the term "proximal" shall mean close to the operator (further away from the target tissue) and "distal" shall mean away from the operator (closer to the target tissue).

The headings provided herein are for convenience only and do not interpret the scope or meaning of the claimed invention.

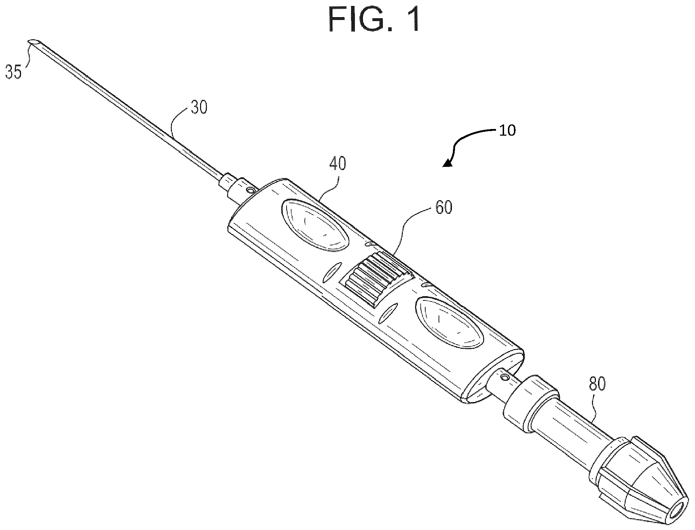

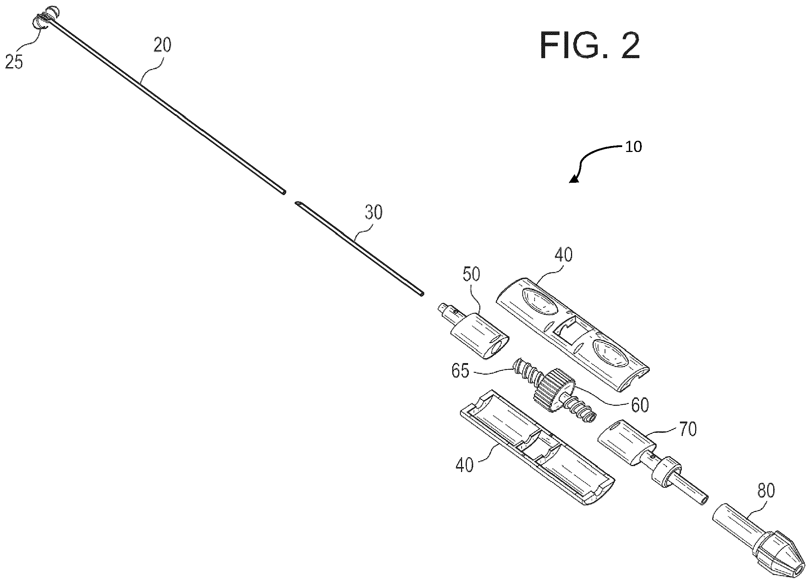

Referring to Figures 1 and 2, an apparatus 10 is shown in the ready to use state. The apparatus 10 comprises a housing 40 having a wheel 60 disposed therein. The housing 40 may have a clamshell design as shown in the figures, however, alternative configurations are possible. For illustrative purposes, in reference to the figures, a threaded rod 65 extends through and engages the center of wheel 60. A first section of threaded rod 65 is threadably engaged with a needle casing 50 and a second section of threaded rod 65 is threadably engaged with anchor casing 70. In some embodiments, the first and second sections of threaded rod 65 are configured as right-handed helix. In some embodiments the first and second sections of the threaded rod 65 are configured as a left-handed helix. In some embodiments, the first section of threaded rod 65 is configured as a right-handed helix and the second section of threaded rod 65 is configured as a left-hand helix, or vice versa. In some embodiments, the first section of threaded rod 65 has a pitch that is equal to or greater than the pitch of the second section of threaded rod 65. In some embodiments, the first section of threaded rod 65 has a pitch that is less than or equal to the pitch of the second section of threaded rod 65. In some embodiments, needle casing 50 and anchor casing 70 are all disposed within housing 40. In other embodiments one or both of needle casing 50 and anchor casing 70 may be disposed either partially or fully external to housing 40. In either configuration, the needle casing 50 and anchor casing 70 are slidable within the housing 40 and, due to relative geometries, are fixed against rotation about the longitudinal axis of housing 40.

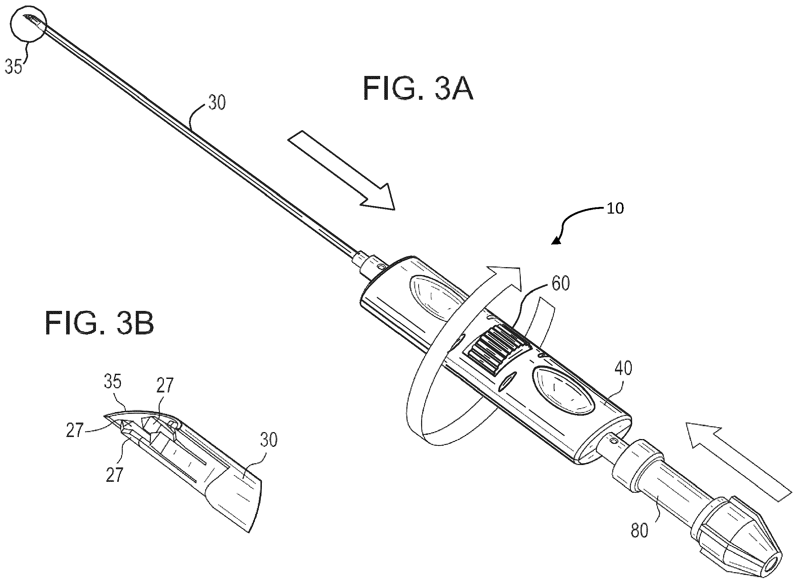

A needle 30 includes a proximal end and a distal end wherein the proximal end is disposed in and securely coupled to needle casing 50. As shown in Figs. 3A and 3B, needle 30 includes a tip 35 having a bevel angle. An anchoring assembly is provided and comprises a tube 20 (see Fig. 2) having an anchoring device 25 attached to the distal end of tube 20 as depicted in Fig. 2. The tube 20 extends through anchor lumens provided in needle 30, needle casing 50 and threaded rod 65, and is disposed in and securely coupled to anchor casing 70.

As shown in Figures 3A-6A, the apparatus according to the invention may be deployed by rotating wheel 60 clockwise, which distally displaces anchor casing 70 as shown in Fig. 2, i.e., draws anchor casing 70 towards wheel 60 as depicted by the arrows in Figs. 3A-6A and which distally displaces tube 20, i.e., pushes tube 20 distally away from wheel 60. Simultaneously, needle casing 50 is proximally displaced, i.e., drawn toward wheel 60, which pulls needle 30 toward wheel 60. The relative movement of needle 30 and tube 20 allows anchor member 25 to deploy from within needle in a uniform and continuous manner and achieve precise positioning. Rotating wheel 60 in the opposite direction, e.g., counter-clockwise, retracts the anchor member 25 back into the tube 20. In alternative embodiments, counter-clockwise rotation of wheel 60 can deploy the anchor member 25, and clockwise rotation of wheel 60 can retract the anchor member 25.

In some embodiments, anchoring device 25 includes a plurality of formed anchoring members 27, e.g., tines or fingers preformed into the shape of a hook and attached to a distal end portion of tube 20. The tines of anchoring member 27 include sharp tips to facilitate tissue penetration, for example into soft tissue. In some embodiments, as best illustrated in Figure 3B, anchoring members 27 are formed at varying positions along and around a circumference of tube 20 to align with the bevel angle of needle tip 35. By align it is meant that in the ready to use state, no portion of anchoring device 25 protrudes past needle tip 35. In some embodiments, each anchoring member 27 includes a proximal end and a distal end wherein the distal end of each member is positioned substantially flush with a circumferential edge of needle tip 35. Anchoring device 25 remains in flush position until wheel 60 is rotated to begin deployment.

In other embodiments, anchoring device 25 may include one or more anchoring members in the form of stacked helical coils, spiral umbrella coils, or the like. In all embodiments, anchoring device 25 may be made of a shape memory material, e.g., Nitinol, or other suitable material.

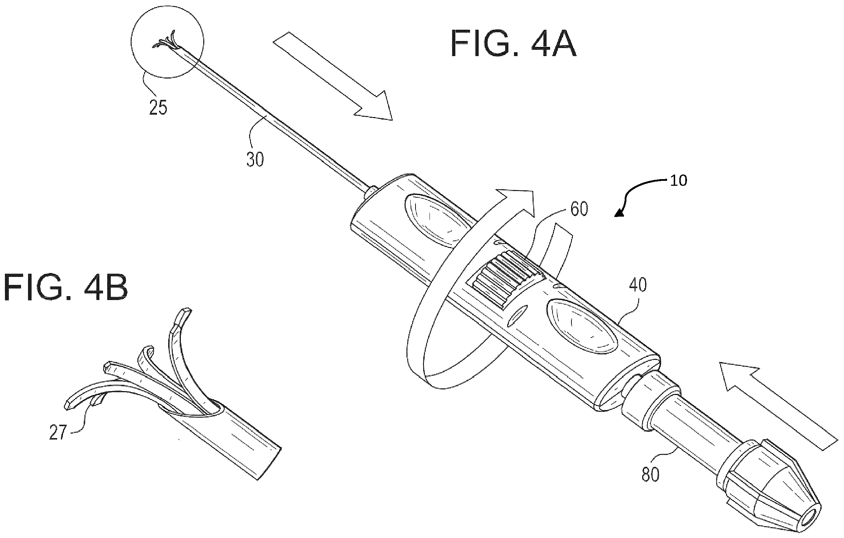

The anchoring device 25 and its associated tines or anchoring members 27 are constrained within needle 30 when the anchoring device 25 is recessed within needle 30, for example, when located in the position shown in Fig. 3B or positions more proximal thereto. The anchoring members 27 expand as the anchoring device 25 is deployed out of the needle 30, for example, by operating wheel 60. Figs. 4A and 4B show anchoring device 25 in a first stage of deployment, where the tines of anchoring members 27 are disposed in a substantially constricted state but are beginning to emerge from needle 30. In this first stage, anchoring members 27 begin to expand radially outward from their position in the constrained configuration and radially outward relative to a longitudinal axis of tube 20 and needle 30 and wherein the end portions of anchoring members 27 are disposed substantially perpendicular to the longitudinal axes of tube 20 and needle 30.

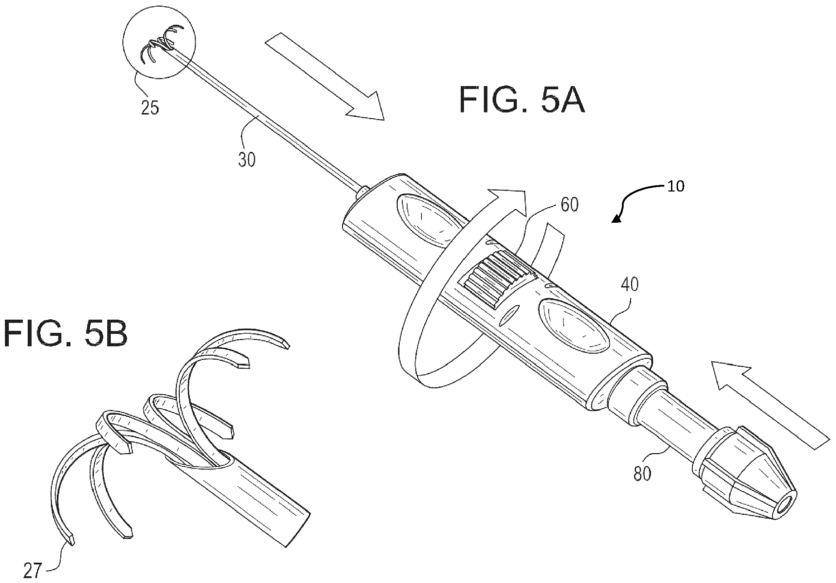

Figs. 5A and 5B depict anchoring device 25 in a second stage of deployment where anchoring members 27 have expanded through the first stage into a C-shape or J-shape configuration where respective end portions of anchoring members are generally parallel with the longitudinal axes of tube 20 and needle 30.

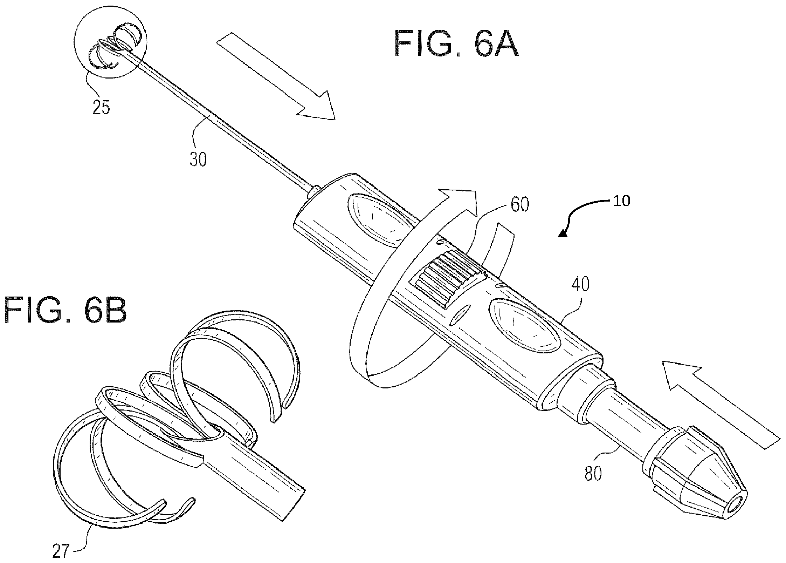

Figs. 6A and 6B illustrate anchoring device 25 fully deployed, for example in a spider leg configuration. By spider leg configuration, it is meant that each anchoring member 27 includes a first section that extends radially outward from the longitudinal axes of needle 30 and tube 20, a second arcuate section, and a third section that extends radially inward towards the longitudinal axes of needle 30 and tube 20..

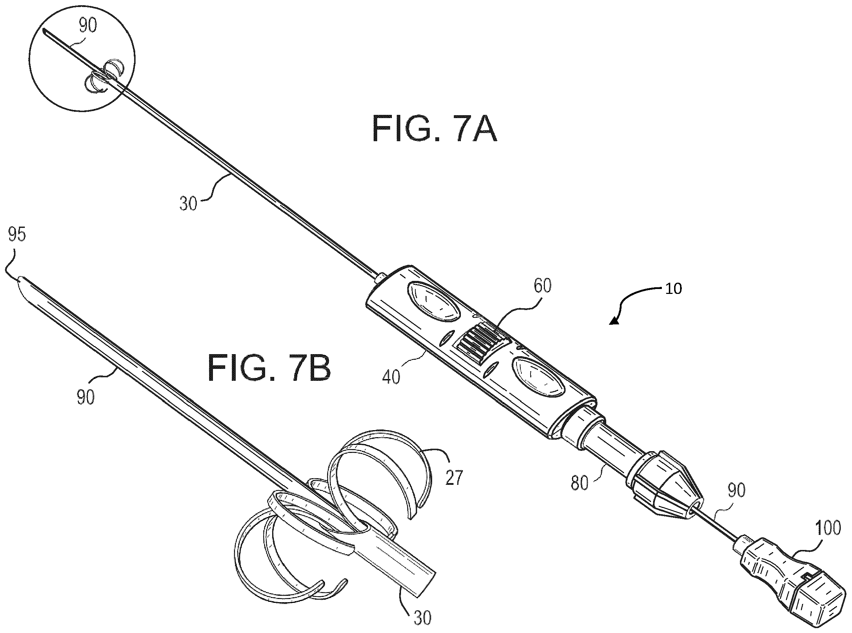

In at least one embodiment, as illustrated in Figures 7A and 7B, the apparatus 10 may be configured to deliver a liquid therapeutic agent into a soft tissue such as a solid tumor. Apparatus 10 may include a fluid delivery assembly having an elongate tubular shaft 90 configured to penetrate into soft tissue. A sharp edge 95 at the distal end of tubular shaft 90 is configured to facilitate penetration into the soft tissue. The sharp edge 95 is beveled and defines a point in this illustrated embodiment, which may help tubular shaft 90 pierce into the soft tissue and be advanced in the soft tissue with a minimal amount of tissue trauma. In one embodiment, the tubular shaft 90 is a needle, such as a hypodermic needle.

In some embodiments, fluid delivery assembly a handle 100 at a proximal end thereof. The handle 100 is configured to facilitate user manipulation of tubular shaft 90. The tubular shaft 90 is configured to move longitudinally relative to needle 30 and tube 20. The handle 100 is configured to be manipulated to cause the longitudinal movement of the tubular shaft 90, e.g., moved in a proximal direction to cause proximal movement of the tubular shaft 90 and moved in a distal direction to cause distal movement of the tubular shaft 90.

The tubular shaft 90 includes an inner lumen (not shown) extending longitudinally therethrough. A liquid therapeutic agent is configured to be passed through the inner lumen and out a distal opening thereof at the distal end of tubular shaft 90 to deliver the liquid therapeutic agent into the soft tissue in which tubular shaft 90 is penetrated.

In some embodiments, tubular shaft 90 has a textured outer surface along at least a partial longitudinal length thereof, e.g., at least along a distal portion of the tubular shaft 90. The textured outer surface of tubular shaft 80 is configured to engage soft tissue when the tubular shaft 90 is penetrated into the soft tissue. The textured outer surface of tubular shaft 90 is configured to increase friction between tubular shaft 90 and the soft tissue to help prevent any movement of the soft tissue relative to tubular shaft 90 and any movement of tubular shaft 90 relative to the soft tissue, which may help ensure that the entire desired amount of the liquid therapeutic agent is delivered into the soft tissue and/or that any fluid gaps around the delivery device 10 (e.g., around the third tube 16) are sealed.

In some embodiments, a locking collet 80 is coupled to anchor casing 70 to lock anchor member 25 in a desired position. Locking anchor member 25 as described may facilitate maintaining anchor member 25 in the optimum configuration. Suitable locking collets include a standard off-the-self torquer or guide wire handle.

A method of using the apparatus described herein is disclosed. The apparatus 10 is introduced into a body of a patient, either directly, e.g., through an incision, or indirectly, e.g., through an access device such as a trocar, cannula, or scoping device. The delivery device 10 is advanced to a target soft tissue in the body of the patient and positioned as desired relative to and outside of the soft tissue. Imaging equipment can be used to assist placement of the delivery device 10 in the desired position. In at least one embodiment, the soft tissue is a solid tumor, the apparatus 10 can be used to deliver a liquid therapeutic agent to other soft tissues. In an exemplary embodiment, the apparatus 10 is in an initial configuration illustrated in Fig. 3B during the introduction of the apparatus 10 into the patient's body and during the delivery device's advancement to and positioning relative to the soft tissue.

With the delivery device 10 in the first configuration and positioned as desired relative to and outside of the soft tissue, the operator rotates wheel 60 which simultaneously draws needle casing 50 and anchor casing 70 toward wheel 60 which distally pushes anchoring assembly 25 causing its sharp ends to expand as illustrated in Figs. 4A, 5A and 6A and penetrate the soft tissue.

The tubular shaft 90 is moved distally relative to the needle 30 and the tube 20 to penetrate the sharp edge 95 of the tubular shaft 90 into the soft tissue. When tubular shaft 90 is in place, a liquid therapeutic agent may be delivered e.g., through the inner lumen the tubular shaft 90, and into the soft tissue.

After delivering the liquid therapeutic agent into the soft tissue, the apparatus 10 may be removed from the patient's body. In some embodiments, anchoring members 27 may be dislodged by rotating wheel 60 in a direction opposite to the direction of rotation from that employed to expand anchoring members 27 as described above. Wheel 60 may be rotated until anchoring members 27 are returned to their initial configuration as depicted in Fig. 3B. In some embodiments tubular member 90 may be removed before either before or after anchoring members 27 are dislodged.

While the foregoing disclosure references particular embodiments of the invention, it will be appreciated by those skilled in the art that changes in these embodiments may be made without departing from the principles of the invention, including embodiments that do not provide all the features and benefits described herein. It will be understood by those skilled in the art that the present disclosure extends beyond the specifically disclosed embodiments to other alternative or additional embodiments and/or uses and obvious modifications and equivalents thereof. In addition, while a number of variations have been shown and described in varying detail, other modifications, which are within the scope of the present disclosure, will be readily apparent to those of skill in the art based upon this disclosure. It is also contemplated that various combinations or subcombinations of the specific features and aspects of the embodiments may be made and still fall within the scope of the present disclosure. Accordingly, it should be understood that various features and aspects of the disclosed embodiments can be combined with or substituted for one another in order to form varying modes of the present disclosure. Thus, it is intended that the scope of the present disclosure herein disclosed should not be limited by the particular disclosed embodiments described above. For all of the embodiments described above, the steps of any methods need not be performed sequentially. The invention is defined by the following claims.

## Claims

1. An apparatus comprising:
a housing (40);
a wheel (60) disposed in and protruding from said housing;
a threaded rod (65) engaged with and attached to said wheel, said threaded rod including a first threaded section and a second threaded section;
a needle casing (50) disposed within said housing, wherein said needle casing being engaged with the first threaded section of said threaded rod;
a needle (30) disposed and secured in said needle casing,
an anchor casing (70) disposed within said housing, wherein said anchor casing being engaged with the second threaded section of said threaded rod; and
anchor assembly including a tube (20) having a proximal end coupled to the anchor casing and distal end including an anchoring device (25), the anchoring device including a plurality of anchor members extending from the proximal end of the tube.

2. The apparatus of claim 1, wherein said anchoring device (25) comprises a shape memory material; optionally wherein the anchor members comprise a shape memory material preformed in the shape of a hook.

3. The apparatus of claim 1 or claim 2, wherein said anchoring device (25) comprises Nitinol.

4. The apparatus of any one of the preceding claims, wherein the needle (30) includes a proximal end and a distal end, the distal end having a bevel angle, optionally wherein one of the following options is fulfilled:
a) the anchoring device includes at least one anchor member aligned with the bevel angle;
b) the anchor members are formed along a circumference of the tube to align with a bevel angle;
c) the anchoring members are attached to and disposed around a circumference of the tube and positioned in alignment with the bevel angle
d) the anchoring members comprise a plurality of fingers, wherein the plurality of fingers are attached to and disposed about the circumference of the tube, each finger including a distal end and a proximal end wherein the distal end of each finger is positioned substantially flush with a circumferential edge of the needle.

5. The apparatus of any one of claims 1 to 4, wherein the first threaded section of said threaded rod (65) includes a right-handed helix and the second threaded section of said threaded rod (65) includes a left-handed helix.

6. The apparatus of any one of claim 1 to 4, wherein the first threaded section of said threaded rod includes a left-handed helix and the second threaded section of said threaded rod includes a right-handed helix.

7. The apparatus of any one of the preceding claims, further comprising a locking collet coupled to said anchor casing (70).

8. The apparatus of any one of the preceding claims, further comprising a fluid delivery assembly including tubular shaft (90) extending distally through said needle and extending proximally through said anchor casing (70), and configured to move axially relative to said needle.

## Patentansprüche

1. Vorrichtung, umfassend:
ein Gehäuse (40);
ein Rad (60), das in dem Gehäuse angeordnet ist und daraus herausragt;
eine Gewindestange (65), die mit dem Rad im Eingriff und daran angebracht ist, wobei die Gewindestange einen ersten Gewindebereich und einen zweiten Gewindebereich aufweist;
ein Nadelgehäuse (50), das in dem Gehäuse angeordnet ist, wobei das Nadelgehäuse mit dem ersten Gewindebereich der Gewindestange im Eingriff ist;
eine Nadel (30), die in dem Nadelgehäuse angeordnet und befestigt ist,
ein Ankergehäuse (70), das in dem Gehäuse angeordnet ist, wobei das Ankergehäuse mit dem zweiten Gewindebereich der Gewindestange im Eingriff ist; und
eine Ankeranordnung mit einer Röhre (20), die ein proximales Ende hat, das mit dem Ankergehäuse gekoppelt ist, und ein distales Ende, das eine Verankerungsvorrichtung (25) aufweist, wobei die Verankerungsvorrichtung eine Vielzahl von Verankerungselementen aufweist, die sich von dem proximalen Ende der Röhre erstrecken.

2. Vorrichtung nach Anspruch 1, wobei die Verankerungsvorrichtung (25) ein Formgedächtnismaterial umfasst, wobei optional die Verankerungselemente ein Formgedächtnismaterial umfassen, das in Form eines Hakens vorgeformt ist.

3. Vorrichtung nach Anspruch 1 oder Anspruch 2, wobei die Verankerungsvorrichtung (25) Nitinol umfasst.

4. Vorrichtung nach einem der vorhergehenden Ansprüche, wobei die Nadel (30) ein proximales Ende und ein distales Ende aufweist, wobei das distale Ende einen Neigungswinkel hat, wobei optional eine der nachstehenden Optionen erfüllt ist:
a) die Verankerungsvorrichtung weist mindestens ein Verankerungselement auf, das auf den Neigungswinkel ausgerichtet ist;
b) die Verankerungselemente sind entlang einem Umfang der Röhre gebildet, um auf einen Neigungswinkel ausgerichtet zu sein;
c) die Verankerungselemente sind an einem Umfang der Röhre angebracht und um diesen angeordnet und in Ausrichtung auf den Neigungswinkel positioniert;
d) die Verankerungselemente umfassen eine Vielzahl von Fingern, wobei die Vielzahl von Fingern an dem Umfang der Röhre angebracht und um diesen angeordnet ist, wobei jeder Finger ein distales Ende und ein proximales Ende aufweist, wobei das distale Ende eines jeden Fingers im Wesentlichen bündig mit einem Umfangsrand der Nadel positioniert ist.

5. Vorrichtung nach einem der Ansprüche 1 bis 4, wobei der erste Gewindebereich der Gewindestange (65) eine rechtsdrehende Spirale und der zweite Gewindebereich der Gewindestange (65) eine linksdrehende Spirale aufweist.

6. Vorrichtung nach einem der Ansprüche 1 bis 4, wobei der erste Gewindebereich der Gewindestange eine rechtsdrehende Spirale und der zweite Gewindebereich der Gewindestange eine linksdrehende Spirale aufweist.

7. Vorrichtung nach einem der vorhergehenden Ansprüche, ferner umfassend einen Sicherungsring, der mit dem Ankergehäuse (70) gekoppelt ist.

8. Vorrichtung nach einem der vorhergehenden Ansprüche, ferner umfassend eine Fluidabgabeanordnung mit einem röhrenförmigen Schaft, der sich distal durch die Nadel erstreckt, und sich proximal durch das Ankergehäuse (70) erstreckt, und dazu ausgelegt ist, sich bezogen auf die Nadel axial zu bewegen.

## Revendications

1. Appareil comprenant :
un boîtier (40) ;
une roue (60) disposée dans ledit boîtier et dépassant de celui-ci ;
une tige filetée (65) en prise avec ladite roue et fixée à celle-ci, ladite tige filetée comprenant une première section filetée et une seconde section filetée ;
une enveloppe d'aiguille (50) disposée à l'intérieur dudit boîtier, ladite enveloppe d'aiguille étant en prise dans la première section filetée de ladite tige filetée ;
une aiguille (30) disposée et fixée dans ladite enveloppe d'aiguille,
une enveloppe d'ancrage (70) disposée à l'intérieur dudit boîtier, ladite enveloppe d'ancrage étant en prise dans la deuxième section filetée de ladite tige filetée ; et
un ensemble d'ancrage comprenant un tube (20) ayant une extrémité proximale couplée à l'enveloppe d'ancrage et une extrémité distale comprenant un dispositif d'ancrage (25), le dispositif d'ancrage comprenant une pluralité d'éléments d'ancrage s'étendant à partir de l'extrémité proximale du tube.

2. Appareil selon la revendication 1, ledit dispositif d'ancrage (25) comprenant un matériau à mémoire de forme ; éventuellement, les éléments d'ancrage comprenant un matériau à mémoire de forme préformé en forme de crochet.

3. Appareil selon la revendication 1 ou selon la revendication 2, ledit dispositif d'ancrage (25) comprenant du Nitinol.

4. Appareil selon l'une quelconque des revendications précédentes, l'aiguille (30) comprenant une extrémité proximale et une extrémité distale, l'extrémité distale ayant un angle de biseau, éventuellement l'une des options suivantes étant remplie :
a) le dispositif d'ancrage comprend au moins un élément d'ancrage aligné avec l'angle de biseau ;
b) les éléments d'ancrage sont formés le long d'une circonférence du tube pour s'aligner sur un angle de biseau ;
c) les éléments d'ancrage sont fixés et disposés autour d'une circonférence du tube et positionnés en alignement avec l'angle de biseau ;
d) les éléments d'ancrage comprennent une pluralité de doigts, la pluralité de doigts étant fixée à la circonférence du tube et disposée autour de celle-ci, chaque doigt comprenant une extrémité distale et une extrémité proximale, l'extrémité distale de chaque doigt étant positionnée sensiblement au même niveau qu'un bord circonférentiel de l'aiguille.

5. Appareil selon l'une quelconque des revendications 1 à 4, la première section filetée de ladite tige filetée (65) comprenant une hélice à droite et la deuxième section filetée de ladite tige filetée (65) comprenant une hélice à gauche.

6. Appareil selon l'une quelconque des revendications 1 à 4, la première section filetée de ladite tige filetée comprenant une hélice à gauche et la deuxième section filetée de ladite tige filetée comprenant une hélice à droite.

7. Appareil selon l'une quelconque des revendications précédentes, comprenant en outre une douille de verrouillage couplée à ladite enveloppe d'ancrage (70).

8. Appareil selon l'une quelconque des revendications précédentes, comprenant en outre un ensemble de distribution de fluide comprenant un arbre tubulaire (90) s'étendant distalement à travers ladite aiguille et s'étendant proximalement à travers ladite enveloppe d'ancrage (70), et configuré pour se déplacer axialement par rapport à ladite aiguille.
